# EUROPEAN PATENT SPECIFICATION

(11) **EP 3 344 230 B1**
(45) Date of publication and mention of the grant of the patent: **06.04.2022**
(21) Application number: 16782325.1
(22) Date of filing: 31.08.2016
(51) Int. Cl.: A61K 9/06, A61K 9/107, A61K 31/135

(54) **FORMULATIONS OF BENZOCAINE LOADED MICROEMULSION BASED HYDROGELS FOR THERAPEUTIC PURPOSES AND THEIR PRODUCTION METHOD**
FORMULIERUNGEN VON BENZOCAINBELADENEN MIKROEMULSIONSBASIERTEN HYDROGELEN FÜR THERAPEUTISCHE ZWECKE UND DEREN HERSTELLUNGSVERFAHREN
FORMULATIONS D'HYDROGELS À BASE DE MICRO-ÉMULSION CHARGÉE DE BENZOCAÏNE À DES FINS THÉRAPEUTIQUES ET LEUR PROCÉDÉ DE PRODUCTION

(30) Priority: 02.09.2015 TR 201510892
(43) Date of publication of application: 11.07.2018
(73) Proprietor: T.C. Istanbul Medipol Universitesi, Beykoz/Istanbul (TR)
(72) Inventor: CAGLAR, Emre Sefik, Istanbul (TR); KARASULU, Hatice Yesim, Izmir (TR); ARPA, Muhammed Davut, Istanbul (TR); USTUDAG OKUR, Neslihan, Istanbul (TR)
(74) Representative: Simsek, Meliha Merve
(86) International application number: PCT/TR2016/050319
(87) International publication number: WO 2017/039559

(56) References cited:
- WO-A1-2008/096351
- NESLIHAN ÜSTÜNDAG OKUR ET AL: "Preparation and Evaluation of Microemulsion Formulations of Naproxen for Dermal Delivery", CHEMICAL AND PHARMACEUTICAL BULLETIN, vol. 62, no. 2, 1 January 2014 (2014-01-01), pages 135-143, XP055319390, JP ISSN: 0009-2363, DOI: 10.1248/cpb.c13-00051
- CHEN H ET AL: "Microemulsion-based hydrogel formulation of ibuprofen for topical delivery", INTERNATIONAL JOURNAL OF PHARMACEUTICS, ELSEVIER, AMSTERDAM, NL, vol. 315, no. 1-2, 6 June 2006 (2006-06-06), pages 52-58, XP025112966, ISSN: 0378-5173, DOI: 10.1016/J.IJPHARM.2006.02.015 [retrieved on 2006-06-06]
- P Uma ET AL: "SELF MICROEMULSIFYING DRUG DELIVERY SYSTEMS: A REVIEW", International Journal of Research in Pharmaceutical and Nano Sciences, 1 January 2013 (2013-01-01), pages 317-331, XP55319422, Retrieved from the Internet: URL:http://www.ijrpns.com/article/Uma Maheswara Reddy P.pdf
- Cristian Peptu ET AL: "Send Orders for Reprints to reprints@benthamscience.ae Nanotechnology Approaches for Pain Therapy Through Transdermal Drug Delivery", Current Pharmaceutical Design, 1 January 2015 (2015-01-01), XP55319406, Retrieved from the Internet: URL:http://www.icmpp.ro/nanoderma/files/Ha rabagiu-MS (1).pdf
- NESLIHAN ÜSTÜNDAG OKUR ET AL: "Preparation and evaluation of novel microemulsion-based hydrogels for dermal delivery of benzocaine", PHARMACEUTICAL DEVELOPMENT AND TECHNOLOGY, 1 January 2016 (2016-01-01), pages 1-11, XP055319389, US ISSN: 1083-7450, DOI: 10.3109/10837450.2015.1131716
- ÜSTÜNDAG-OKUR NESLIHAN ET AL: "Preparation andin vitro-in vivoevaluation of ofloxacin loaded ophthalmic nano structured lipid carriers modified with chitosan oligosaccharide lactate for the treatment of bacterial kerat", EUROPEAN JOURNAL OF PHARMACEUTICAL SCIENCES, ELSEVIER AMSTERDAM, NL, vol. 63, 8 August 2014 (2014-08-08), pages 204-215, XP029056597, ISSN: 0928-0987, DOI: 10.1016/J.EJPS.2014.07.013
- Turkey Erzincan: "INTERNATIONAL EURASIA PHARMACY CONGRESS", Thursday, 3 September 2015 (2015-09-03), pages 3-720, XP055319418, Retrieved from the Internet: URL:http://erpharmacy.erzincan.edu.tr/wp-c ontent/uploads/2015/09/28.8.2015EN-SON-Rev ize-Son-kongre-program-tablo-1.pdf [retrieved on 2016-11-15]
- Mitra Jelvehgari ET AL: "Mucoadhesive and Drug Release Properties of Benzocaine Gel", Iranian Journal of Pharmaceutical Sciences Autumn, 1 January 2006 (2006-01-01), XP055319177, Retrieved from the Internet: URL:http://www.ijps.ir/article_1945_988553 a735143686dd84fc8a81b76b51.pdf

## Description

### Technical Field

This invention is related to preparation of microemulsion based drug carrier system which its viscosity improved by adding gelling agents for the anesthetic purposes.

This invention is especially related to preparation of microemulsion formulations involving benzocaine as a local anesthetic active ingredient and latter, to achievement of microemulsion based hydrogels by converting microemulsions into gels using gelling agents.

### Previous Technical

Dermal drug delivery has been used for a long time since skin is easy to access, has a large surface area with vast exposure to the circulatory and lymphatic networks and the route is noninvasive. Transdermal and topical administration of drugs has major advantages compare to classical oral administration of the drugs. The advantages of dermal systems are suitability, enhanced patient compliance and elimination of hepatic first-pass effect.

Local anesthetics are a class of drugs able to induce pain relief by virtue of their ability to bind to the sodium channel of excitable membranes, thus blocking the influx of sodium ions and the conduction of the nervous impulse. Benzocaine, or paraamino benzoic acid ethyl ester (molecular formula C₉H₁₁NO₂, molecular weight 165.189 g/mol), is a local anesthetic used primarily to relieve pain or irritations on the skin and mucosal surface. It is one of the ester types of long-acting local anesthetics, which has been used for the relief of local pain.

In current technology, several different techniques were developed providing absorption of drugs through skin. Some of the techniques being used today are formulations which are being administered over the skin surface such as creams and gels. During treatment, high viscosity systems such as creams and gels are localized longer over the skin surface. However, they need to interact with skin for the therapeutic effect at least for 1 hour. In addition, the administration site should be covered after application. These systems are not preferred due to unwanted conditions such as the risk of late therapeutic effect of the formulation and of contagion of the formulation to the patients' outfit. Formulations in solution form are more administrable rather than these systems but preparing solution formulation of benzocaine is hard due to its low water solubility.

The promising technique among currently available ones is microemulsions.

Microemulsions are thermodynamically stable, isotropically clear drug delivery systems that have a droplet size smaller than 0.15 µm. It is very well known that microemulsions carry more drugs through skin layers faster than the other drug carrier systems such as solutions and creams. Microemulsions consist of oil, surfactant, cosurfactant and water. They have several pharmaceutical advantages, such as ease of preparation, transparency and potentials for solubilizing variety of drugs. In addition to these, they are suitable vehicles for dermal and transdermal delivery of drugs. The components of microemulsions reduce the diffusion barrier effect of skin by interacting with stratum corneum. They provide increased concentration and thermodynamic activity of drugs at the site of application, and hydration effect on the stratum corneum. The most important disadvantage of microemulsions which have numerous advantages is hardness of their stabilization at the application site because of their low viscosity..

In current technique, the other drug delivery system under use is hydrogels. These systems can easily remove the disadvantages of microemulsions and localize on the skin surface longer due to their high viscosity. Although this advantage of these systems, the main disadvantage of hydrogels are their inability to go through the skin layers

The applications that we came across during our literature search are:
In the state of technique no: 6,074,674 in American Patent document, local anesthetic formulation of continued-prolonged released microcrystal and its preparation method by using wax and non-ionic polymers is being expressed.

The other one of these techniques is the application no. WO2014123100 of which describes the development of lidocaine and benzocaine loaded local anesthetic patches.

The other one of these techniques is the application no. JP2004149544. In this document, preparation of some anesthetic substances loaded microemulsions by using ingredients such Lutrol^{™} F68 and Lutrol^{™} F127 and preparation methodology of formulations which converts into gels at the administration site by adding thermosensitive gelling agents into these microemulsions have been explained. The thermosensitive gel formulation described in this document is convenient for intraoral usage but administration of this formulation on skin surface is not a proper way. Because prepared formulations are liquids and they gels at body temperature. Due to these formulations would not stay on skin surface for enough time, preparing thermosensitive gel would not be beneficial for dermal application.

Similarly the other application is no: CA2627021A1. This application is about gel formulations in which benzocaine and various herbs such as Hamamelis virginiana are combined. In several researches, gel formulations of benzocaine studied in order to extend the localization time of benzocaine on skin surface. However, stratum corneum which localized at the first layer of the skin behaves as barrier and limits the drug transportation through skin's layers. Therefore it decreases the bioavailability of the drug.

In another study which is titled as 'Preparation and evaluation of bioadhesive benzocaine gels for enhanced local anesthetic effects' (2003) written by Shin SC, Lee JW, Yang KH, Lee CH, benzocaine loaded bioadhesive gel formulations were prepared by using hydroxypropyl methylcellulose (1).

In the article 'Mucoadhesive and Drug Release Properties of Benzocaine Gel' (2006) written by Jelvehgari M, Rashidi MR, Samadi H, (2) the preparation of benzocaine loaded gel formulations by using Carbopol^{™} was explained. Once these articles were read, it can easily be seen that both gel formulations which are prepared by using either Cabopol^{™} or HPMC (hydroxypropyl methylcellulose) are prolonging the duration of active substance on the skin surface but could not provide enough amount of active substance and long duration of permeation through skin layers. This exhibits a disadvantage situation.

In another study titled as New "drug-in cyclodextrin-in deformable liposomes" formulations to improve the therapeutic efficacy of local anesthetics' (3) performed by Maestrelli F, Gonzalez-Rodriguez ML, Rabasco AM, Ghelardini C, Mura P.; it is explained that preparation of benzocaine loaded liposomal gel formulation by using HPβCD (hydroxypropyl-β-cyclodextrine). The expense of liposome technology and their stability problems play main disadvantage role for prepared liposomal gel formulations.

The other similar one of these is American patent application no. US8652088. This application is about a preparation method of some drug substances loaded microemulsions by using microdialysis technique. In this study, microemulsion is the only one formulation prepared. In terms of administering microemulsion to skin, low viscosity of microemulsions plays a disadvantageous role.

In previous technique another practice (as stated in the article Development, characterization and in vivo evaluation of benzocaine-loaded liposomes' written by Mura P, Maestrelli F, Gonzalez-Rodriguez ML, Michelacci I, Ghelardini C, Rabasco AM) explains the preparation method of benzocaine loaded liposomal gel formulation (4). The main disadvantages of these types of formulations are expensive ingredients and a need for special devices in terms of preparing liposomal formulations.

Other prior art which are of relevence to the present invention are listed below;
- Neslihan Ustundag Okur et. al:"Preparation and Evaluation of Microemulsion Formulations of Naproxen for Dermal Delivery" Chemical and Pharmaceutical Bulletin, vol 62, no. 2, 1 January 2014, pages 135-143 is disclosing a Naproxen formulation for topical application compare said microemulsion formulation with the commercial formulation. In this document use of a combination of surfactants such as Labrafil^{™}, Labrasol^{™} and Span^{™} 80 are used to increase retention time of microemulsions.
- Chen H. et al: Microemulsion-based hydrogel formulation of ibuprofen for topical delivery" International Journal of Pharmaceutics, vol 315, no. 1-2, 6 June 2006, pages 52-58 is directed to microemulsion-based hydrogel formulations for Ibuprofen wherein Xanthan gum is used as the gel matrix in order to improve the viscosity of the microemulsion.
- P Uma et. el, "Self emulsifying drug delivery systems: A review" International Journal of Research in Pharmaceutical and Nano Sciences, 1 January 2013, pages 317-331 is in the general field of oral microemulsions and specifically it is directed to self emulsifying drug delivery systems, the document has no information regarding microemulsions for use in topical delivery of benzocaine.
- WO 2008/096351 A1 is disclosing transdermal or transmucosal pharmaceutical compositions suitable for extra-vascular application of an active substance. This application is teaching the use of propylene carbonate to enhance the bioavailability of biologically active substance.
- Cristian Peptu et. al: "nanotechnology Approaches for Pain Therapy Through Transdermal Drug Delivery", Current Pharmaceutical Design, 1 January 2015, this publication is a review of the research carried out in the area of pain treatment via transdermal drug delivery. The paper is especially focusing on the formulations for enhancement of the penetration of anesthetics wherein the drug molecule is encapsulated in macrocyclic molecules such as cyclodextrins and their derivatives or liposomes or polymer nanoparticles or hydrogels.

As conclusion, due to problematic situations mentioned above and inadequate solutions to these problematic conditions, development in technical field for drug delivery systems is necessitated.

### The Aim of the Invention

Current invention is about benzocaine loaded microemulsion based hydrogels for the purpose of treatment and their preparation method which meets all the requirements and removes all the disadvantageous situations and provides advantageous conditions..

Prior aim of the invention is preparation of local anesthetic benzocaine loaded microemulsion formulations, afterwards, increasing viscosity of microemulsion formulations by adding gelling agents in terms of having prolonged release of these formulations and converting them into microemulsion based hydrogels.

One of the purposes of this invention is preparation microemulsion based hydrogel systems which combine microemulsion systems and hydrogel systems in order to achieve extended localization on skin due to their high viscosity and to transfer drugs through skin's layers.

The other aim of this invention is to regulate the release rate of benzocaine and prolong its anesthetic effect by using microemulsion based hydrogel system. By this means, duration of localization on skin surface is prolonged and systemic toxicity is decreased.

Another purpose of the invention is to increase drug permeation by virtue of the fact that microemulsion based hydrogel systems decreases the barrier effect of skin. Because of this feature of microemulsion based hydrogels, these systems are more than appropriate in terms of administering drug transdermal or dermal.

Similar aim of this invention is to add gelling agents into microemulsion formulations for prolonging the duration of the stay on the skin surface and therefore providing long therapeutic effect of drug substance.

In order to fulfil the purposes mentioned above, steps of preparation method of microemulsion based hydrogels are listed:
a) Preparation of microemulsion formulations,
   - Mixing the ratio in between 0,5-5% w/v of benzocaine with the ratio of 1-25% w/v of liquid lipid which is isopropyl myristate
   - Adding 5-30% w/v of individual or combination of surfactant(s) Span^{™} 20, Polisorbate 80, Tween^{™} 20, Cremophor^{™} EL and Cremophor^{™} RH40.
   - Adding ethanol into this system as cosurfactant with the ratio of 5-30% w/v and then stirring.
   - Adding the ratio of 5-80% w/v of distilled water into mixture and stirring.
   - Stirring prepared microemulsions,
b) Adding the ratio in between 0,1-15 % w/v of Carbopol^{™} into microemulsion formulations as gelling agent and then stirring.

### Figures Ease the Understandability of Invention

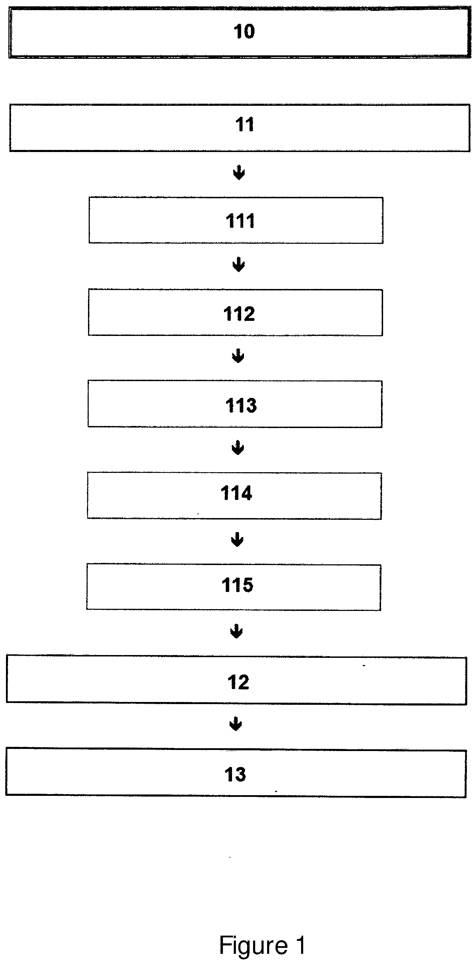

**Figure 1****:** Production flow diagram of invention which is microemulsion based hyrogels.

### Explanation of References in Figure

10. Production method of microemulsion based hydrogels
11. Preparation of microemulsion formulations
   111. Mixing anesthetic ingredient with liquid lipid
   112. Adding surfactants into previous mixture mentioned above
   113. Adding co-surfactant ingredient into previous mixture and then stirring
   114. Adding distilled water into previous mixture and stirring
   115. Stirring microemulsion which is recently formed
12. Adding gelling agents into prepared microemulsion formulations
13. Adding triethanolamine into this mixture in terms of formation of gels.

### Detailed description of the invention

In this detailed description, the invention which is benzocaine loaded microemulsion based hydrogel formulations and the preferred structure of preparation method of these formulations will be described with no limitations for better understanding of the subject.

The invention is about preparation of microemulsion formulations involving benzocaine which is local anesthetic drug substance and latter, obtainment microemulsion based hydrogels by converting these microemulsions into gels with help of added gelling agent.

Microemulsion based hydrogel which is the invention subject is a topically administrable therapeutic gel involving microemulsion structure that is convenient for industrial application, able to decrease the release rate of active ingredient and thus able to localize longer on/in skin surface and its layers.

Benzocaine is one of the anesthetic drugs which is used in terms of removing unwanted pain and relieving skin burnsand injuries. This is the reason why benzocaine needs to be rapidly acted and localize at the administration site longer in order to prolong the duration of its strength.

Microemulsion based hydrogel formulations decrease the barrier effect of the skin and thus increase the permeation of the drug through skin layers. This is the reason why these formulations are very convenient structures for dermal and transdermal administration of the drug. The substances forming the formulations are harmless and rapid acting agents at the administration site because of being chosen as proper for dermal usage. In addition, according to microscopic studies, it has been shown that they were transported effectively through skin layers in an hour.

Therefore, in current technique, benzocaine loaded microemulsion based hydrogel systems provide longer stay on skin surface.

### The invention subject benzocaine loaded microemulsion based hydrogel formulations and their preparation method involves the steps below:

- Preparation of microemulsion formulations (11),
   - mixing anesthetic substance with liquid lipid (111),
   - Adding surfactant substances into previous mixture (112),
   - adding cosurfactant substance into previous mixture and stirring (113),
   - adding distilled water into previous mixture and stirring (114),
   - Stirring prepared microemulsions for a significant time (in current invention, it is 5 minutes) (115),
- Adding gelling agents into prepared microemulsions and stirring in order to obtain microemulsion based hydrogel (12),
- Adding triethanolamine into the prepared mixture in order to make the formulation gel (13),

In the method of invention (10), at first glance, microemulsions which provide nano scale structure for final products are prepared (11). For this, anesthetic substance is being mixed with liquid lipid (111). In an application of the invention, isopropyl myristate is used as liquid lipid and benzocaine is used as anesthetic substance. The isopropyl myristate is a strong permeation enhancer and increase the diffusion coefficient of skin.

In addition, the pseudoternary-phase diagrams are constructed by using high concentration of surfactant and co-surfactant for the preparation of microemulsions. The pseudoternary-phase diagrams are necessary in order to obtain the microemulsion area. In this invention, five different surfactants are used. Surfactants are added into this mixture at the same temperature (112). In an application of this invention, Span^{™} 20, polysorbate 80 (Tween^{™} 80), Tween^{™} 20, Cremophor^{™} EL and Cremophor^{™} RH40 is used as surfactants individually or with a combination. The combination ratios of surfactants are; 1:1, 1:2, 1:3, 1:4, 1:5, 1:6, 1:7, 1:8, 1:9, 2:1, 3:1, 4:1, 5:1, 6:1, 7:1, 8:1, 9:1.

Afterwards, cosurfactant substances are added into these mixtures (113). In an application of invention, ethanol is used as cosurfactant. As final step, distilled water is added into the mixture and is stirred (114). In an application of the invention, distilled water is used. Afterwards, microemulsions are stirred for five minutes (115).

The ratios by weight / volume of materials used in preparation of benzocaine loaded microemulsions (11) are: 1- 25% w/v liquid lipid, 0.5 - 5% w/v anesthetic substance, 5 - 30% w/v surfactant, 5 - 30% w/v cosurfactant and 5 - 80% distilled water to complete 100%.

In the next step, gelling agents are added into the prepared microemulsions (12). In an application of the invention, gelling agent Carbopol^{™} is added as 0.1 - 15 % w/v of the mixture (12). By doing so, microemulsion based hydrogels are obtained.

The invention subject microemulsion based hydrogel are obtained by stirring the mixture. The biocompatible gelling agent extends the residence time of the invention subject microemulsion based hydrogel formulations on the skin surface and thus increases the efficacy of the treatment.

The invention is very suitable for industrial manufacturing. It can be produced by the pharmaceutical companies which manufacture the semi solid systems. Materials and equipments for preparation procedure are very easy to access. The devices in use for preparation of formulations are simple stirrers which every company already possesses.

### REFERENCES

1. Shin SC, Lee JW, Yang KH, Lee CH, Preparation and evaluation of bioadhesive benzocaine gels for enhanced local anesthetic effects, International Journal of Pharmaceutics, 260, 77-81, 2003.
2. Jelvehgari M, Rashidi MR, Samadi H, Mucoadhesive and Drug Release Properties of Benzocaine Gel, Iranian Journal of Pharmaceutical Sciences, 2(4), 185-194, 2006.
3. Maestrelli F, Gonzalez-Rodriguez ML, Rabasco AM, Ghelardini C, Mura P, New "drug-in cyclodextrin-in deformable liposomes" formulations to improve the therapeutic efficacy of local anaesthetics, International Journal of Pharmaceutics, 395, 222-231, 2010.
4. Mura P, Maestrelli F, Gonzalez-Rodriguez ML, Michelacci I, Ghelardini C, Rabasco AM, Development, characterization and in vivo evaluation of benzocaine-loaded liposomes European Journal of Pharmaceutics and Biopharmaceutics. 67(1):86-95, 2007.

## Claims

1. Preparation method of benzocaine loaded microemulsion based hydrogel (10)
a) Preparation of microemulsion formulations (11),
• Mixing 0.5 - 5 %w/v benzocaine with 1 - 25% w/v liquid lipid which is isopropyl myristate (111),
• Adding 5 - 30% w/v surfactant itself or combinations of surfactants which are Span^{™} 20, Polysorbate 80, Tween^{™} 20, Cremophor^{™} EL and Cremophor^{™} RH40 (112),
• Adding 5 - 30% w/v ethanol as cosurfactant into this mixture and stirring. (113),
• Stirring the mixture by adding 5 - 80% w/v distilled water (114),
• Stirring the prepared microemulsions (115),
b) Adding 0,1 - 15% w/v gelling agent Carbopol^{™} into prepared microemulsion formulations and stirring (12),

2. Preparation method which is convenient to Claim 1 (10); Stirring microemulsions, which are prepared according to procedure step a, for five minutes

3. Preparation method which is convenient to Claim 1 (10); after the procedure step b, adding triethanolamine into the mixture in order to obtain the gel and stirring (13)

4. Preparation method which is convenient to Claim 3 (10); the concentration ratio of the triethanolamine is in between 0.1 - 5 % w/v.

## Patentansprüche

1. Verfahren zur Herstellung eines mit Benzocain beladenen, auf Mikroemulsion basierenden Hydrogels (10)
a) Herstellung von Mikroemulsionsformulierungen (11),
• Mischen von 0.5 - 5 % Gew./Vol. Benzocain mit 1 - 25 % Gew./Vol. flüssigem Lipid, das Isopropylmyristat ist (111),
• Zusetzen von 5 - 30 % Gew./Vol. Tensid selbst oder Kombinationen von Tensiden wie Span^{™} 20, Polysorbat 80, Tween^{™} 20, Cremophor^{™} EL und Cremophor^{™} RH40 (112),
• Zusetzen 5-30 % Gew./Vol. Ethanol als Co-Tensid in diese Mischung und Rühren (113),
• Rühren der Mischung durch Zusetzen von 5 - 80 % Gew./Vol. destilliertem Wasser (114),
• Rühren der hergestellten Mikroemulsionen (115),
b) Zusetzen von 0,1 - 15% Gew./Vol. des Geliermittels Carbopol^{™} in hergestellte Mikroemulsionsformulierungen und Rühren (12),

2. Verfahren zur Herstellung nach Anspruch 1 (10); Rühren von Mikroemulsionen, die gemäß Verfahrensschritt a hergestellt wurden, für fünf Minuten

3. Verfahren zur Herstellung nach Anspruch 1 (10); nach dem Verfahrensschritt b, Zusetzen von Triethanolamin in die Mischung, um das Gel zu erhalten, und Rühren (13)

4. Verfahren zur Herstellung nach Anspruch 1 (10); das Konzentrationsverhältnis von Triethanolamin beträgt zwischen 0.1 - 5 % Gew./Vol.

## Revendications

1. Procédé de préparation d'un hydrogel à base de microémulsion chargé de benzocaïne (10)
a) Préparer des formulations en microémulsion (11),
• Mélanger 0.5 - 5 % p/v de benzocaïne avec 1 - 25 % p/v de lipide liquide qui est le myristate d'isopropyle (111),
• Ajouter 5 - 30% p/v de tensioactif lui-même ou des combinaisons de tensioactifs qui sont Span^{™} 20, Polysorbate 80, Tween^{™} 20, Cremophor^{™} EL et Cremophor^{™} RH40 (112),
• Ajouter 5 - 30 % p/v d'éthanol comme cosurfactant dans ce mélange et agiter (113),
• Agiter le mélange en ajoutant 5 - 80 % p/v d'eau distillée (114),
• Agiter les microémulsions préparées (115),
b) Ajouter 0,1 - 15% p/v d'agent gélifiant Carbopol^{™} dans les formulations de microémulsion préparées et agiter (12).

2. Procédé de préparation selon la revendication 1 (10); Agiter les microémulsions, qui sont préparées selon l'étape a du procédé, pendant cinq minutes

3. Procédé de préparation selon la revendication 1 (10); après l'étape b de la procédure, ajouter de la triéthanolamine dans le mélange afin d'obtenir le gel et agiter (13)

4. Procédé de préparation selon la revendication 3 (10); le rapport de concentration de la triéthanolamine est compris entre 0.1 - 5 % p/v.
